(19) Europäisches Patentamt
European Patent Office
Office européen des brevets

(11) EP 2 934 433 B1

(12) **EUROPEAN PATENT SPECIFICATION**

(45) Date of publication and mention of the grant of the patent:
**02.02.2022 Bulletin 2022/05**

(21) Application number: **13866763.9**

(22) Date of filing: **18.12.2013**

(51) International Patent Classification (IPC):
***A61K 8/27*** (2006.01) ***A61K 8/28*** (2006.01)
***A61K 8/29*** (2006.01) ***A61K 8/58*** (2006.01)
***A61K 8/63*** (2006.01) ***A61Q 17/04*** (2006.01)
***A61Q 19/00*** (2006.01) ***A61K 8/891*** (2006.01)
***A61Q 1/02*** (2006.01) ***A61Q 19/08*** (2006.01)

(52) Cooperative Patent Classification (CPC):
**A61K 8/891; A61K 8/27; A61K 8/28; A61K 8/29; A61K 8/58; A61K 8/63; A61Q 1/02; A61Q 17/04; A61Q 19/00; A61Q 19/08**

(86) International application number:
**PCT/CN2013/089862**

(87) International publication number:
**WO 2014/101700 (03.07.2014 Gazette 2014/27)**

(54) **COSMETIC COMPOSITION**

KOSMETISCHE ZUSAMMENSETZUNG

COMPOSITION COSMÉTIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **24.12.2012 PCT/CN2012/087301**
**01.02.2013 EP 13153662**

(43) Date of publication of application:
**28.10.2015 Bulletin 2015/44**

(73) Proprietors:
- **Unilever Global IP Limited**
  **Wirral, CH62 AZD (GB)**
  Designated Contracting States:
  **CY DE GB IE IT MT**
- **Unilever IP Holdings B.V.**
  **3013 AL Rotterdam (NL)**
  Designated Contracting States:
  **AL AT BE BG CH CZ DK EE ES FI FR GR HR HU IS LI LT LU LV MC MK NL NO PL PT RO RS SE SI SK SM TR**

(72) Inventors:
- **QIU, Qiang**
  **Shanghai 200335 (CN)**
- **WANG, Xiuxia**
  **Shanghai 200335 (CN)**

(74) Representative: **James, Helen Sarah et al**
**Unilever PLC**
**Unilever Patent Group**
**Bronland 14**
**6708 WH Wageningen (NL)**

(56) References cited:
**EP-A2- 1 772 138** **EP-A2- 2 263 639**
**WO-A1-03/026596** **WO-A1-2005/107691**
**KR-A- 20080 086 620** **US-A1- 2008 292 560**
**US-A1- 2010 080 768** **US-A1- 2013 028 955**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 934 433 B1

## Description

### FIELD OF THE INVENTION

The present invention relates to a cosmetic composition. Particularly, the present invention relates to a cosmetic composition comprising a film-forming polymer having a contact angle of at least 85°, a steroid, and cosmetically acceptable carrier, wherein (i) the weight ratio of film-forming polymer to steroid is at least 5:9; (ii) the carrier is water; (iii) the film-forming polymer is silicone resin; and (iv) the amount of water is 20 to 95% by weight of the composition. The composition is useful for topical application to skin, in particular for forming a film with improved benefits of skin firming, wash-off resistance, abrasion resistance, cumulative deposition of beneficial agent and/or long-lasting deposition of beneficial agent.

### BACKGROUND OF THE INVENTION

Usually, consumers have some skin problems including dryness, wrinkles and fine lines, loose/saggy skin and age spots. Cosmetic composition comprising film-forming polymer may be one solution for consumers to these problems. Film-forming polymer would form a film onto the skin after applying topically and bring immediate firming effect to the skin. Some beneficial agents, for example optical particle and sunscreen agent, may also be delivered onto skin surface together with the film-forming polymer.

There is an increasing interest to develop a skin care composition comprising a film-forming polymer.

US patent application with publication number of US 2008/0233075 A1 disclosed a topical composition comprising a water-soluble film-forming polymer, a bimodal copolymer comprising a first polymeric component with anionic functional groups and a second polymeric component with cationic functional groups, and one or more biological polymers that are derived from a source selected from the group consisting of animals, plants, algae, fungi, and bacteria or are biotechnologically synthesized. Such a topical composition was said to be applied to saggy or wrinkled skin for enhancing the appearance of the skin.

European patent application with publication number of EP 1172138 A2 discloses an organopolysiloxane combination for surface treating powder, powder treated with the combination and a cosmetic comprising the powder. The cosmetic comprising the powder is said to have good affinity to the skin or hair and lasts long on the skin or hair.

However, after applying cosmetic composition, the skin may undergo water washing and abrasion by hand and therefore the film formed by film-forming polymer on the skin may be easily washed away and/or rubbed away and therefore lose the firming benefits. Meanwhile, the beneficial agent would be easily washed off and/or rubbed off and thus can not provide a long-lasting benefit.

Therefore, the present inventors have recognized a need to develop a cosmetic composition with improved wash-off resistance, abrasion resistance, and/or long-lasting deposition of beneficial agent. Therefore, this invention is directed to a cosmetic composition comprising a film-forming polymer having a contact angle of at least 85°, a steroid, and cosmetically acceptable carrier, wherein (i) the weight ratio of film-forming polymer to steroid is at least 5:9; (ii) the carrier is water; (iii) the film-forming polymer is silicone resin; and (iv) the amount of water is 20 to 95% by weight of the composition. It was surprisingly found that such a cosmetic composition may improve the performance of skin firming, wash-off resistance, abrasion resistance, cumulative deposition of beneficial agent and/or long-lasting deposition of beneficial agent.

### DEFINITIONS

#### Silicone resin

The term "silicone resin" as used herein refers to silicone material which is formed by branched, and/or cage-like oligosiloxanes having three-dimensional structure. Typically, the silicone resin is rigid.

#### Leave-on and Wash-off

The term "leave-on" as used with reference to compositions herein means a composition that is applied to or rubbed on the skin, and left thereon.

The term "wash-off" as used with reference to compositions herein means a skin cleanser that is applied to or rubbed on the skin and rinsed off substantially immediately subsequent to application.

Viscosity

Viscosity for the purposes of the present invention means kinematic viscosity at 25°C and is reported as centiStokes (1 cSt = 1 $mm^2 \cdot s^{-1}$). Viscosity of fluids such as silicone oil can be determined, for example, by the relevant international standard, such as ISO 3104.

Refractive Index

Refractive index values referred to herein are those determined at a temperature of 25 °C and a wavelength of 589 nm unless otherwise stated.

Skin

The term "skin" as used herein includes the skin on the face (except eye lids and lips), neck, chest, abdomen, back, arms, hands, and legs. Preferably "skin" means skin on the face.

Film-forming polymer

"Film-forming polymer" as used herein refers to polymer which is capable of forming cohesive and continuous covering over the hair and/or skin when applied to their surface.

Contact angle

Contact angle, as used herein, means the angle at which a water/vapor interface meets a solid surface at a temperature of 25°C. Such an angle may be measured with a goniometer or other water droplet shape analysis systems with water droplet of 5 μl and at 25°C.

Miscellaneous

Except in the examples, or where otherwise explicitly indicated, all numbers in this description indicating amounts of material or conditions of reaction, physical properties of materials and/or use may optionally be understood as modified by the word "about".

All amounts are by weight of the final composition, unless otherwise specified.

It should be noted that in specifying any range of values, any particular upper value can be associated with any particular lower value.

For the avoidance of doubt, the word "comprising" is intended to mean "including" but not necessarily "consisting of" or "composed of'. In other words, the listed steps or options need not be exhaustive.

The disclosure of the invention as found herein is to be considered to cover all embodiments as found in the claims as being multiply dependent upon each other irrespective of the fact that claims may be found without multiple dependency or redundancy.

Where a feature is disclosed with respect to a particular aspect of the invention (for example a composition of the invention), such disclosure is also to be considered to apply to any other aspect of the invention (for example a method of the invention) *mutatis mutandis.*

**SUMMARY OF THE INVENTION**

In a first aspect, the present invention directed to a cosmetic composition comprising film-forming polymer having a contact angle of at least 85°, a steroid, and cosmetically acceptable carrier, wherein (i) the weight ratio of film-forming polymer to steroid is at least 5:9; (ii) the carrier is water; (iii) the film-forming polymer is silicone resin; and (iv) the amount of water is 20 to 95% by weight of the composition.

In a second aspect, the present invention directed to a method for improving skin characteristics selected from skin firming, wash-off resistance, abrasion resistance, cumulative deposition of beneficial agent, long-lasting beneficial agent deposition wherein "long-lasting" refers to the beneficial agent remains at least 30% after flushing by tap water (25°C) for 1 minute, comprising the step of topically applying to skin the composition of any one of the preceding claims, wherein the beneficial agent comprises optical particle, sunscreen agent or a mixture thereof.

In a third aspect, the present invention directed to use of any embodiment of the first aspect for improving any attribute selected from skin firming, wash-off resistance, abrasion resistance, cumulative deposition of beneficial agent, beneficial agent deposition wherein "long-lasting" refers to the beneficial agent remains at least 30% after flushing by

tap water (25°C) for 1 minute, or combination thereof, wherein the beneficial agent comprises optical particle, sunscreen agent or a mixture thereof.

All other aspects of the present invention will more readily become apparent upon considering the detailed description and examples which follow.

## DETAILED DESCRIPTION

The requirement for film-forming polymer is that the film-forming polymer is suitable to be employed in cosmetic composition. For better performance of wash-off resistance, the film-forming polymer preferably has a contact angle of at least 90°, more preferably from 95° to 160°, most preferably from 100° to 120°.

The film-forming polymer is silicone resin.

Preferably, the film-forming polymer is present in the composition in amount of from 0.01 to 20% by weight of the composition, more preferably from 0.2 to 10 %, even more preferably from 0.5 to 7%, and most preferably from 1 to 4% by weight of the composition.

The weight ratio of film-forming polymer to steroid is at least 5:9. For better performance of wash-off resistance, abrasion resistance and/or long-lasting benefits of beneficial agent, the weight ratio of film-forming polymer to steroid is preferably from 5:7 to 50:1, more preferably from 3:2 to 35:1, more preferably still from 9:4 to 20:1 even more preferably from 5:2 to 8:1 and most preferably from 8:3 to 5:1.

The present invention provides a composition a cosmetic composition comprising a silicone resin, a steroid, and cosmetically acceptable carrier, wherein the weight ratio of silicone resin to steroid is at least 5:9.

The limitation of the silicone resin of the present invention is that the silicone resin is suitable to be used in cosmetic composition and the silicone resin is a film-forming silicone resin. The silicone resin is typically described by the following siloxy monomeric units:

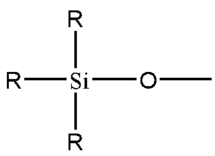
$M=(R)_3SiO_{1/2}$

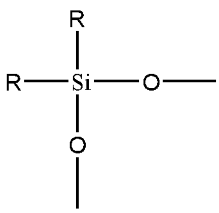
$D=(R)_2SiO_{2/2}$

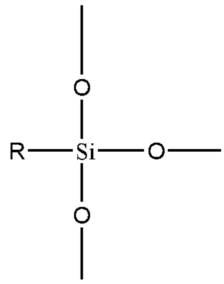
$T=RSiO_{3/2}$

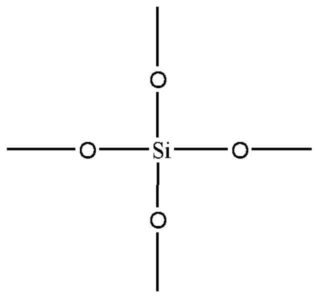
$Q=SiO_{4/2}$

The R group may be selected from saturated or unsaturated hydrocarbon groups. Preferably, the silicone resin of the present invention may be selected from siloxysilicate, silsesquioxane, or a mixture thereof. More preferably, the silicone resin comprises M unit, Q unit, T unit or combination thereof. Even more preferably, the silicone resin comprises MQ silicone resin, T silicone resin, or a mixture thereof.

In some embodiments, the silicone resin preferably comprises MQ silicone resin having the formula of $[(R_1)_3\text{-Si-}O_{1/2}]_a\text{-}(Si\text{-}O_{4/2})_b$, wherein $R_1$ is mutually identical or different, selected from saturated hydrocarbon groups. $R_1$ is preferably selected from $C_1$ to $C_6$ alkyl, and more preferably each $R_1$ is methyl group. Thus, the more preferred MQ silicone resin is trimethylsiloxysilicate. Preferably, a and b independently have values ranging from 10 to 1000, and more preferably from 30 to 200.

In another embodiments, the silicone resin preferably comprises T silicone resin having the formula of $[R_2\text{-Si-}O_{3/2}]_x$, wherein $R_2$ is selected from saturated hydrocarbon groups. $R_2$ is preferably selected from $C_1$ to $C_6$ alkyl, more preferably selected from methyl, ethyl, propyl, butyl, and most preferably propyl. The most preferred T silicone resin is polypropyl silsesquioxane. Preferably, x is less than 2000, more preferably less than 500, but preferably greater than 10, and more preferably greater than 50.

In certain embodiments, the silicone resin preferably comprises a blend of MQ silicone resin and T silicone resin, the weight ratio of the MQ silicone resin to the T silicone resin is preferably from 1:20 to 20:1 in order to achieve better film-forming performance. More preferably, the weight ratio of the MQ silicone resin to the T silicone resin is from 1:10 to 10:1, even more preferably from 1:5 to 5:1.

In most preferred embodiments, the composition comprises MQ silicone resin. Preferably the MQ silicone resin is present in amount of at least 20% by weight of the total silicone resin, more preferably at least 40%, even more preferably at least 70%, and still even more preferably at least 85% by weight of the total silicone resin. Most preferably,

MQ silicone resin is present in amount of 100% by weight of the total silicone resin.

Exemplary silicone resin suitable for the present invention includes Dow Corning™ MQ-1640 Flake Resin, a blend of MQ and T Propyl resins, Dow Corning™ MQ-1600 Solid Resin, a 100% active MQ resin, Dow Corning™ 670 Fluid, Cyclopentasiloxane (and) Polypropylsilsesquioxane supplied by Dow Corning.

Preferably, the silicone resin is present in the composition in amount of from 0.01 to 20% by weight of the composition, more preferably from 0.2 to 10%, even more preferably from 0.5 to 7%, and most preferably from 1 to 4% by weight of the composition.

There is no particular limitation regarding to the steroid of the present invention provided that the steroid is suitable to use in cosmetic composition. Preferably, the steroid comprises hydroxyl group, more preferably comprise a sterol and even more preferably the steroid is a sterol. The sterol may comprise phytosterol, zoosterol, fungi sterol, or a mixture thereof. It is preferred that the sterol comprise zoosterol.

Exemplary sterol includes cholesterol, β-sitosterol, stigmasterol, campersterol, brassicasterol, ergosterol, cholestanol, cholestenone, 7-ketocholesterol, 5α,6α-epoxycholestanol, 5β,6β epoxycholestanol, and 7-dehydrocholesterol, 15-ketocholestene, 15-ketocholestane, 25-hydroxycholesterol, 27-hydroxycholesterol, 24-hydroxycholesterol, 24,25-epoxycholesterol, 24-dihydrolanosterol, lanosterol, or a mixture thereof

It is preferred that the sterol comprises cholesterol, oxysterol, or a mixture thereof. More preferably the sterol comprises cholesterol. The sterol preferably comprises at least 30% of cholesterol by weight of the sterol, more preferably at least 50% by weight, and even more preferably from 80 to 100% by weight of the sterol. Most preferably, the sterol is cholesterol.

The steroid is preferably present in the composition in amount of from 0.01 to 10% by weight of the composition, more preferably from 0.05 to 5%, even more preferably from 0.1 to 3%, and most preferably from 0.3 to 1.5% by weight of the composition.

The weight ratio of silicone resin to steroid is at least 5:9. For better performance of wash-off resistance, abrasion resistance and/or long-lasting benefits of beneficial agent, the weight ratio of silicone resin to steroid is preferably from 5:7 to 50:1, more preferably from 3:2 to 35:1, more preferably still from 9:4 to 20:1 even more preferably from 5:2 to 8:1 and most preferably from 8:3 to 5:1.

Compositions of the present invention will also include a cosmetically acceptable carrier. Water is the carrier. Amounts of water range from 20 to 95%, more preferably from 40 to 90%, optimally between 60 and 85% by weight of the cosmetic composition.

Emollient materials may be included as carriers in compositions of this invention. These may be in the form of silicone oils, synthetic esters and/or hydrocarbons. Amounts of the emollients may range, for example, anywhere from 0.1 to 95%, more preferably between 1 and 50% by weight of the composition.

Silicone oils may be divided into the volatile and nonvolatile variety. The term "volatile" as used herein refers to those materials which have a measurable vapor pressure at ambient temperature (25 °C). Volatile silicone oils are preferably chosen from cyclic (cyclomethicone) or linear polydimethylsiloxanes containing from 3 to 9, preferably from 4 to 5, silicon atoms. In many liquid versions of compositions according to the present invention, the volatile silicone oils may form a relatively large component of the compositions as carriers. Amounts may range, for example, from 5% to 80%, more preferably from 20% to 70% by weight of the composition.

Nonvolatile silicone oils useful as an emollient material include polyalkyl siloxanes, polyalkylaryl siloxanes and polyether siloxane copolymers. The essentially nonvolatile polyalkyl siloxanes useful herein include, for example, polydimethyl siloxanes with viscosities of from about $5 \times 10^{-6}$ to 0.1 $m^2/s$ at 25 °C. Among the preferred nonvolatile emollients useful in the present compositions are the polydimethyl siloxanes having viscosities from about $1 \times 10^{-5}$ to about $4 \times 10^{-4}$ $m^2/s$ at 25 °C.

Organopolysiloxane crosspolymers can be usefully employed. Representative of these materials are dimethicone/vinyl dimethicone crosspolymers and dimethicone crosspolymers available from a variety of suppliers including Dow Corning (9040, 9041, 9045, 9506 and 9509), General Electric (SFE 839), Shin Etsu (KSG-15, 16 and 18 [dimethicone/phenyl vinyl dimethicone crosspolymer]), and Grant Industries (Gransil brand of materials), and lauryl dimethicone/vinyl dimethicone crosspolymers supplied by Shin Etsu (e.g. KSG-31, KSG-32, KSG-41, KSG-42, KSG-43 and KSG-44). Amounts of the aforementioned silicone elastomers (when present) will usually be from 0.1 to 20% by weight dissolved usually in a volatile silicone oil such as cyclomethicone.

When silicones are present in large amounts as carrier and water is also present, the systems may be oil continuous. These normally will require emulsification with a water-in-oil emulsifier such as a dimethicone copolyol (e.g. Abil EM-90 which is cetyl dimethicone copolyol).

Among the ester emollients are:

a) Alkenyl or alkyl esters of fatty acids having 10 to 20 carbon atoms. Examples thereof include isoarachidyl neopentanoate, isodecyl neopentanoate, isononyl isonanoate, cetyl ricinoleate, oleyl myristate, oleyl stearate, and oleyl oleate.

b) Ether-esters such as fatty acid esters of ethoxylated fatty alcohols.

c) Polyhydric alcohol esters. Butylene glycol, ethylene glycol mono and di-fatty acid esters, diethylene glycol mono- and di-fatty acid esters, polyethylene glycol (200-6000) mono- and di-fatty acid esters, propylene glycol mono- and di-fatty acid esters, polypropylene glycol 2000 monooleate, polypropylene glycol 2000 monostearate, ethoxylated propylene glycol monostearate, glyceryl mono-and di-fatty acid esters, polyglycerol poly-fatty esters, ethoxylated glyceryl mono-stearate, 1,3-butylene glycol monostearate, 1,3-butylene glycol distearate, polyoxyethylene polyol fatty acid ester, sorbitan fatty acid esters, and polyoxyethylene sorbitan fatty acid esters are satisfactory polyhydric alcohol esters. Particularly useful are pentaerythritol, trimethylolpropane and neopentyl glycol esters of $C_1$-$C_{30}$ alcohols. Exemplative is pentaerythrityl tetraethylhexanoate.

d) Wax esters such as beeswax, spermaceti wax and tribehenin wax.

e) Sterols esters, of which cholesterol fatty acid esters are examples thereof.

f) Sugar ester of fatty acids such as sucrose polybehenate and sucrose polycottonseedate.

Of particular use also are the $C_{12-15}$ alkyl benzoate esters sold under the Finsolve brand.

Hydrocarbons which are suitable cosmetically acceptable carriers include petrolatum, mineral oil, $C_{11}$-$C_{13}$ isoparaffins, polyalphaolefins, and especially isohexadecane, available commercially as Permethyl 101A from Presperse Inc.

Humectants of the polyhydric alcohol-type can be employed as cosmetically acceptable carriers. Typical polyhydric alcohols include polyalkylene glycols and more preferably alkylene polyols and their derivatives, including propylene glycol, dipropylene glycol, polypropylene glycol, polyethylene glycol and derivatives thereof, sorbitol, hydroxypropyl sorbitol, hexylene glycol, 1,3-butylene glycol, isoprene glycol, 1,2,6-hexanetriol, glycerol, ethoxylated glycerol, propoxylated glycerol and mixtures thereof. The amount of humectant may range, for example, anywhere from 0.5 to 50%, more preferably between 1 and 15% by weight of the composition. Most preferred is glycerol (also known as glycerin). Amounts of glycerin may range, for example, from 1% to 50%, more preferably from 10 to 35%, optimally from 15 to 30% by weight of the composition.

Preferably the cosmetic composition comprises particles which impart opacity to skin, hereafter term "optical particles". Without being bound to any particular theory or explanation, the present inventors believe that optical particles would be embedded into the film by silicone resin and steroid. Therefore, the optical particles are able to resist water and/or friction and deliver the long-lasting opacity to the skin.

The optical particles are typically particles of high refractive index materials. For example the optical particles may have a refractive index of greater than 1.3, more preferably greater than 1.7 and most preferably from 2.0 to 2.7. Examples of such optical particles are those comprising bismuth oxychloride, boron nitride, barium sulfate, mica, silica, titanium dioxide, zirconium oxide, iron oxide, aluminium oxide, zinc oxide or combinations thereof. Even more preferred are particles comprising zinc oxide, zirconium oxide, titanium dioxide or a combination thereof as these materials have especially high refractive index. Most preferred is titanium dioxide.

For sake of good compatibility with the film-forming polymer and/or cholesterol, the optical particle is preferably hydrophobic. More preferably, the optical particle is preferably hydrophobically modified. Even more preferably the optical particle is modified by hydrophobic material selected from fatty acid, silicone oil, wax, and a mixture thereof. The fatty acid preferably comprises oleic acid, stearic acid, or a mixture thereof.

Preferably the composition comprises optical particles in an amount of from 0.001 to 10 wt%, more preferably 0.01 to 7 wt%, more preferably still 0.05 to 5 wt% and most preferably 0.1 to 2 wt%.

The weight ratio of the film-forming polymer to the optical particle is preferably in the range of from 1:10 to 50:1, more preferably from 1:3 to 10:1, and most preferably from 1:1 to 5:1. The weight ratio of the steroid to the optical particle is preferably in the range of from 1:40 to 20:1, more preferably from 1:20 to 10:1, and most preferably from 1:10 to 5:1.

When the composition comprises silicone resin and optical particle, the weight ratio of the silicone resin to the optical particle is preferably in the range of from 1:10 to 50:1, more preferably from 1:3 to 10:1, and most preferably from 1:1 to 5:1.

Besides optical particles, the compositions of this invention may include a variety of other functional ingredients. Sunscreen actives may be included in compositions of the present invention. These will be organic compounds having at least one chromophoric group absorbing within the ultraviolet ranging from 290 to 400 nm. Chromophoric organic sunscreen agents may be divided into the following categories (with specific examples) including: p-Aminobenzoic acid, its salts and its derivatives (ethyl, isobutyl, glyceryl esters; p-dimethylaminobenzoic acid); Anthranilates (o-aminobenzoates; methyl, menthyl, phenyl, benzyl, phenylethyl, linalyl, terpinyl, and cyclohexenyl esters); Salicylates (octyl, amyl, phenyl, benzyl, menthyl, glyceryl, and dipropyleneglycol esters); Cinnamic acid derivatives (menthyl and benzyl esters,

alpha-phenyl cinnamonitrile; butyl cinnamoyl pyruvate); Dihydroxycinnamic acid derivatives (umbelliferone, methylumbelliferone, methylaceto-umbelliferone); Trihydroxycinnamic acid derivatives (esculetin, methylesculetin, daphnetin, and the glucosides, esculin and daphnin); Hydrocarbons (diphenylbutadiene, stilbene); Dibenzalacetone and benzalacetophenone; Naphtholsulfonates (sodium salts of 2-naphthol-3,6-disulfonic and of 2-naphthol-6,8-disulfonic acids); Dihydroxy-naphthoic acid and its salts; o- and p-Hydroxybiphenyldisulfonates; Coumarin derivatives (7-hydroxy, 7-methyl, 3-phenyl); Diazoles (2-acetyl-3-bromoindazole, phenyl benzoxazole, methyl naphthoxazole, various aryl benzothiazoles); Quinine salts (bisulfate, sulfate, chloride, oleate, and tannate); Quinoline derivatives (8-hydroxyquinoline salts, 2-phenylquinoline); Hydroxy- or methoxy-substituted benzophenones; Uric and vilouric acids; Tannic acid and its derivatives (e.g., hexaethylether); (Butyl carbityl) (6-propyl piperonyl) ether; Hydroquinone; Benzophenones (Oxybenzone, Sulisobenzone, Dioxybenzone, Benzoresorcinol, 2,2',4,4'-Tetrahydroxybenzophenone, 2,2'-Dihydroxy-4,4'-dimethoxybenzophenone, Octabenzone; 4-Isopropyldibenzoylmethane; Butylmethoxydibenzoylmethane; Etocrylene; and 4-isopropyl-dibenzoylmethane). Particularly useful are: 2-ethylhexyl p-methoxycinnamate, 4,4'-t-butyl methoxydibenzoylmethane, 2-hydroxy-4-methoxybenzophenone, octyldimethyl p-aminobenzoic acid, digalloyltrioleate, 2,2-dihydroxy-4-methoxybenzophenone, ethyl 4-[bis(hydroxypropyl)]aminobenzoate, 2-ethylhexyl-2-cyano-3,3-diphenylacrylate, 2-ethylhexylsalicylate, glyceryl p-aminobenzoate, 3,3,5-trimethylcyclohexylsalicylate, methylanthranilate, p-dimethylaminobenzoic acid or aminobenzoate, 2-ethylhexyl p-dimethylaminobenzoate, 2-phenylbenzimidazole-5-sulfonic acid, 2-(p-dimethylaminophenyl)-5-sulfoniobenzoxazoic acid and mixtures thereof.

Particularly preferred are such materials as ethylhexyl p-methoxycinnamate, available as Parsol MCX®, Avobenzone, available as Parsol 1789®, Dermablock OS® (octylsalicylate) and Mexoryl SX® (with INCI name of Terephthalylidene Dicamphor Sulfonic Acid).

Amounts of the organic sunscreen agent may range, for example, from 0.1 to 15%, more preferably from 0.5% to 10%, optimally from 1% to 8% by weight of the composition.

A variety of thickening agents may be included in the compositions. Illustrative but not limiting are stearic acid, Acrylamide/Sodium Acryloyldimethyltaurate Copolymer (Aristoflex AVC), Hydroxyethyl Acrylate/Sodium Acryloyldimethyltaurate Copolymer, Aluminum Starch Octenyl Succinate, Polyacrylates (such as Carbomers including Carbopol® 980, Carbopol® 1342, Pemulen TR-2® and the Ultrez® thickeners), Polysaccharides (including xanthan gum, guar gum, pectin, carageenan and sclerotium gums), celluloses (including carboxymethyl cellulose, ethyl cellulose, hydroxyethyl cellulose and methyl hydroxymethyl cellulose), minerals (including talc, silica, alumina, mica and clays, the latter being represented by bentonites, hectorites and attapulgites), magnesium aluminum silicate and mixtures thereof. Amounts of the thickeners may range, for example, from 0.05 to 10%, more preferably from 0.3 to 2% by weight of the composition.

Preservatives can desirably be incorporated into the cosmetic compositions of this invention to protect against the growth of potentially harmful microorganisms. Suitable traditional preservatives for compositions of this invention are alkyl esters of para-hydroxybenzoic acid. Other preservatives which have more recently come into use include hydantoin derivatives, propionate salts, and a variety of quaternary ammonium compounds. Cosmetic chemists are familiar with appropriate preservatives and routinely choose them to satisfy the preservative challenge test and to provide product stability. Particularly preferred preservatives are phenoxyethanol, methyl paraben, propyl paraben, butyl paraben, isobutyl paraben, imidazolidinyl urea, sodium dehydroacetate and benzyl alcohol. The preservatives should be selected having regard for the use of the composition and possible incompatibilities between the preservatives and other ingredients in the composition. Preservatives are preferably employed in amounts ranging from 0.01% to 2% by weight of the composition.

Compositions of the present invention may also contain vitamins and flavonoids. Illustrative water-soluble vitamins are Niacinamide, Vitamin $B_2$, Vitamin $B_6$, Vitamin C and Biotin. Among the useful water-insoluble vitamins are Vitamin A (retinol), Vitamin A Palmitate, ascorbyl tetraisopalmitate, Vitamin E (tocopherol), Vitamin E Acetate and DL-panthenol. A particularly suitable Vitamin $B_6$ derivative is Pyridoxine Palmitate. Among the preferred flavonoids are glucosyl hesperidin and rutin. Total amount of vitamins or flavonoids when present in compositions according to the present invention may range, for example, from 0.001 to 10%, more preferably from 0.01% to 1%, optimally from 0.1 to 0.5% by weight of the composition.

Desquamation agents are further optional components. Illustrative are the alpha-hydroxycarboxylic acids and beta-hydroxycarboxylic acids and salts of these acids. Among the former are salts of glycolic acid, lactic acid and malic acid. Salicylic acid is representative of the beta-hydroxycarboxylic acids. Amounts of these materials when present may range from 0.1 to 15% by weight of the composition.

A variety of herbal extracts may optionally be included in compositions of this invention. Illustrative are pomegranate, white birch (Betula Alba), green tea, chamomile, licorice, boswellia serrata, olive (Olea Europaea) leaf, arnica montana flower, lavandula angustifolia, and extract combinations thereof.

The extracts may either be water soluble or water-insoluble carried in a solvent which respectively is hydrophilic or hydrophobic. Water and ethanol are the preferred extract solvents.

Miscellaneous other adjunct cosmetic ingredients that may be suitable for the present compositions include ceramides (e.g. Ceramide 3 and Ceramide 6), conjugated linoleic acids, colorants (e.g. iron oxides), metal (manganese,

copper and/or zinc) gluconates, allantoin, palmitoyl pentapeptide-3, amino acids (e.g. alanine, arginine, glycine, lysine, proline, serine, threonine, glumatic acid and mixtures thereof), trimethylglycine, sodium PCA, chelator like disodium EDTA, opacifiers like titanium dioxide, magnesium aspartate, and combinations thereof. Amounts may, for example, vary from 0.000001 to 3% by weight of the composition.

A small amount of emulsifying surfactant may be present. Surfactants may be anionic, nonionic, cationic, amphoteric and mixtures thereof. Levels may range, for example, from 0.1 to 5%, more preferably from 0.1 to 2%, optimally from 0.1 to 1% by weight. Advantageously the amount of surfactant present should not be sufficient for lather formation. In these instances, less than 2% by weight, preferably less than 1%, and optimally less than 0.5% by weight surfactant is present. Emulsifiers like PEG-100 stearate may be used as well as emulsion stabilizers like cetearyl alcohol and ceteareth-20 may be used and typically in amounts that do not exceed 5 percent by weight of the composition.

Other optional additives suitable for use in the composition of this invention include cationic ammonium compounds to enhance moisturization. Such compounds include salts of hydroxypropyltri ($C_1$-$C_3$ alkyl) ammonium mono-substituted-saccharide, salts of hydroxypropyltri ($C_1$-$C_3$ alkyl) ammonium mono-substituted polyols, dihydroxypropyltri ($C_1$-$C_3$ alkyl) ammonium salts, dihydroxypropyldi ($C_1$-$C_3$ alkyl) mono(hydroxyethyl) ammonium salts, guar hydroxypropyl trimonium salts, 2,3-dihydroxypropyl tri($C_1$-$C_3$ alkyl or hydroxalkyl) ammonium salts or mixtures thereof. In a most preferred embodiment and when desired, the cationic ammonium compound employed in this invention is the quaternary ammonium compound 1,2-dihydroxypropyltrimonium chloride. If used, such compounds typically make up from 0.01 to 30%, and more preferably from about 0.1 to about 15% by weight of the composition.

When cationic ammonium compounds are used, optional additives for use with the same are moisturizing agents such as substituted ureas like hydroxymethyl urea, hydroxyethyl urea, hydroxypropyl urea; bis(hydroxymethyl) urea; bis(hydroxyethyl) urea; bis(hydroxypropyl) urea; N,N'-dihydroxymethyl urea; N,N'-di-hydroxyethyl urea; N,N'-di-hydroxypropyl urea; N,N,N'-tri-hydroxyethyl urea; tetra(hydroxymethyl) urea; tetra(hydroxyethyl) urea; tetra(hydroxypropyl) urea; N-methyl-N'-hydroxyethyl urea; N-ethyl-N'-hydroxyethyl urea; N-hydroxypropyl-N'-hydroxyethyl urea and N,N'dimethyl-N-hydroxyethyl urea or mixtures thereof. Where the term hydroxypropyl appears, the meaning is generic for either 3-hydroxy-n-propyl, 2-hydroxy-n-propyl, 3-hydroxy-i-propyl or 2-hydroxy-i-propyl radicals. Most preferred is hydroxyethyl urea. The latter is available as a 50% aqueous liquid from AkzoNobel under the trademark Hydrovance. Such substituted ureas, while desirable in moisturizing formulations, are only selected for use when compatible with sunless tanning agent or agents (when used) in the compositions of this invention.

Amounts of substituted urea, when used, in the composition of this invention range from 0.01 to 20%, more preferably from 0.5 to 15%, and most preferably from 2 to 10% based on total weight of the composition and including all ranges subsumed therein.

When cationic ammonium compound and substituted urea are used, in a most especially preferred embodiment at least from 0.01 to 25%, more preferably from 0.2 to 20%, and most preferably from 1 to 15% humectant, like glycerine, is used, based on total weight of the composition and including all ranges subsumed therein.

When making the compositions of this invention, ingredients are typically mixed with moderate shear under atmospheric conditions. The compositions may be applied topically and preferably 1-4 milligrams of composition is applied per square centimeter of skin. Preferably, the compositions display a pH from 5 to 7. Packaging for the composition of this invention can be ajar or tube as well as any other format typically seen for cosmetic, cream, washing and lotion type products.

It is preferred that the composition is a skin care composition. The composition may be a leave-on composition or a wash-off composition, but preferably a leave-on composition.

The invention also concerns a method for improving skin characteristics comprising the step of topically applying to skin the cosmetic composition of the invention as described. Skin characteristics as used herein refers to features used to evaluate skin, include but not limit to skin firming, opacity, smoothness, cleanliness, moistening, or a combination thereof. Preferably, the skin characteristics comprise skin firming, opacity, or a combination thereof. More preferably skin characteristic is long-lasting opacity. Opacity as used herein often includes masking/reducing blemishes, even skin tone and/or skin lightening

The cosmetic composition of the invention is intended to use for improving any attribute selected from skin firming, wash-off resistance, abrasion resistance, long-lasting beneficial agent deposition, cumulative deposition of beneficial agent, or combination thereof. "Long-lasting" refers to the beneficial agent (for example optical particle) remains at least 30%, preferably at least 50% after flushing by tap water (25°C) for 1 minute. The beneficial agent comprises optical particle, sunscreen agent or a mixture thereof. More preferably, the beneficial agent is optical particle.

The following examples are provided to facilitate an understanding of the invention. The examples are not intended to limit the scope of the claims.

## EXAMPLES

Wash-off/abrasion resistance test

1. Constructing a calibration curve

The base formulation (sample A in Table 1) was coated evenly onto Bio-skin plate (Color: 30#, ex. BEAULAX, Co. Ltd., Tokyo, Japan) with surface density of 0, 0.5, 1, 1.5, 2, 2.5, 3, 3.5, 4, 5, and 6 mg/cm$^2$. After naturally drying at around 25°C for 8 hours, the lightness of these coated Bio-skin plates was measured using Digieye Imaging System (Verivide, UK). Then the lightness versus surface density was plotted and fitted by a 4 orders' polynomial model to obtain the curve. The R-Square was higher than 0.999 which demonstrated that the polynomial model was suitable to fit the functional relationship between the lightness and the surface density of the base formulation.

2. Wash-off/abrasion experiment

30 mg of samples was coated evenly onto Bio-skin plates with area of 10 cm$^2$. The coated Bio-skin plate was naturally dried at around 25°C for 8 hours. The lightness of the Bio-skin plate ($L_1$) was measured by Digieye Imaging System (Verivide, UK). The surface density value before wash-off/abrasion experiment ($SD_1$) was obtained according to the calibration curve. The coated bio-skin was soaked into de-ionized water for 30s. Then, a commercial face cleanser (Pond's gold radiance™ Radiance Boosting Cleansing Mousse) with amount of 5 mg/cm$^2$ was applied onto the Bio-skin plate and the coated Bio-skin plate was washed by Martindale abrasion and pilling tester (Type: M235, SDL Altas, USA) with 33.72g of motion plate at the speed of 30 rpm for 1 min. Subsequently, the coated Bio-skin plate was soaked into water for another 1 min and washed by de-ionized water. After naturally drying at around 25°C for 2 hours, the lightness of the Bio-skin plate ($L_2$) was measured again by Digieye Imaging System. The surface density after wash-off/abrasion experiment ($SD_2$) was obtained according to the calibration curve.
The deposition ratio after wash-off/abrasion experiment was calculated by:

$$\text{Deposition ratio} = (SD_2/SD_1)\times 100\%.$$

Measurement of Contact Angle and water soaking test

The contact angles of five film-forming polymers including Dow Corning™ MQ-1640 Flake Resin, Dow Corning™ MQ-1600 Solid Resin, Dow Corning™ 670 Fluid from Dow Corning, Avalure™ UR450 from Lubrizol, Lexorez™ 100 from Inolex were conducted. Dow Corning™ MQ-1600 Solid Resin and Dow Corning™ 670 Fluid were dispersed into dimethicone with a polymer to solvent weight ratio of 1:9. The other three polymers were dispersed into ethanol with a polymer to solvent weight ratio of 1:9.
0.2 ml of film-forming polymer dispersions were dripped evenly onto an ordinary glass sides (about 2 cm×8cm). After the solvents evaporated, uniform films were formed. Drop shape analysis system 100 (DSA 100, Krüss) was used to measure contact angle using deionised water drops of around 5 μL applied to five different points of each film. The contact angle averaged over all 5 drops.
The contact angles of Dow Corning™ MQ-1640 Flake Resin, Dow Corning™ MQ-1600 Solid Resin, Dow Corning™ 670, Avalure™ UR450, and Lexorez™ 100 were 107°, 116°, 114°, 66°, and 28° respectively.
Then, these glass slides coated with polymer films were immersed fully into water for 30 minutes. After that the glass slides were taken out from water and dried. The contact angles of these glass slides were measured again. It was surprisingly found that the contact angles of glass slides coated by Avalure™ UR450, and Lexorez™ 100 were decreased to less than 15°, indicating that the polymer film has been peeled off. In contrast, the contact angle of glass slides coated by Dow Corning™ MQ-1640 Flake Resin, Dow Corning™ MQ-1600 Solid Resin, and Dow Corning™ 670 remained almost the same, manifesting that the polymer films were adhered onto the glass slides firmly.

Example 1

This example demonstrates the inclusion of cholesterol into the composition with different silicone resin improved the film wash-off resistance, abrasion resistance and therefore deposition of beneficial agent.
A series of cosmetic compositions were formulated as shown in Table 1 below. The formulations were prepared by the following process. The optical particles were completely dispersed in the oil phase with other ingredients and the silicone resin and/or cholesterol (when present) and mixed thoroughly. The resulting oil-based mixture was gradually added to the aqueous phase. The resulting mixture was emulsified under 9,000 rpm of shear stress for 10 minutes at

65 °C and gradually stirred and cooled to room temperature.

The deposition ratios of the samples were measured by following the *Wash-off/abrasion resistance performance test.*

Table 1

| Ingredient (wt%) | Sample | | | | | | | |
|---|---|---|---|---|---|---|---|---|
| | A | B | C | D | E | 1 | 2 | 3 |
| Water | Bal. | Bal. | Bal. | Bal. | Bal. | Bal. | Bal. | Bal. |
| Disodium EDTA | 0.10 | 0.10 | 0.10 | 0.10 | 0.10 | 0.10 | 0.10 | 0.10 |
| PEG-100 Stearate (Myij 59 P) | 1.85 | 1.85 | 1.85 | 1.85 | 1.85 | 1.85 | 1.85 | 1.85 |
| Glyceryl stearate | 1.00 | 1.00 | 1.00 | 1.00 | 1.00 | 1.00 | 1.00 | 1.00 |
| Caprylic / Capric Triglycerides | 3.00 | 3.00 | 3.00 | 3.00 | 3.00 | 3.00 | 3.00 | 3.00 |
| Aristoflex AVC UL | 1.00 | 1.00 | 1.00 | 1.00 | 1.00 | 1.00 | 1.00 | 1.00 |
| Cyclomethicone/DC 245 | 8.00 | 8.00 | 8.00 | 8.00 | 8.00 | 8.00 | 8.00 | 8.00 |
| Preservative | 0.20 | 0.20 | 0.20 | 0.20 | 0.20 | 0.20 | 0.20 | 0.20 |
| Titanium dioxide[a] | 1.00 | 1.00 | 1.00 | 1.00 | 1.00 | 1.00 | 1.00 | 1.00 |
| Silicone resin-1 [b] | --- | 3.00 | --- | --- | --- | 3.00 | --- | --- |
| Silicone resin-2 [c] | --- | --- | 3.00 | --- | --- | --- | 3.00 | --- |
| Silicone resin-3 [d] | --- | --- | --- | 3.00 | --- | --- | --- | 3.00 |
| Cholesterol [e] | --- | --- | --- | --- | 1.00 | 1.00 | 1.00 | 1.00 |

a. MT700Z, Titanium Dioxide (and) Stearic Acid (and) Aluminium Hydroxide supplied by TAYCA
b. Dow Corning™ MQ-1640 Flake Resin, a blend of MQ and T Propyl resins, supplied by Dow Corning.
c. Dow Corning™ MQ-1600 Solid Resin, a 100% active MQ resin, supplied by Dow Corning.
d. Dow Corning™ 670 Fluid (45% active), Cyclopentasiloxane (and) Polypropylsilsesquioxane supplied by Dow Corning.
e. Cholesterol NF supplied by RITA corporation.

As can be seen in the Table 2, the compositions comprising different kinds of silicone resin alone (samples B, C and D) had a deposition ratio of over or around 35%. After inclusion of cholesterol into the samples, the deposition ratio of all the samples (Sample 1, 2 and 3) were not only increased, but also greater than the sum of deposition ratio of composition comprising silicone resin alone and composition comprising cholesterol alone (sample E). It was manifested that the addition of cholesterol into composition comprising silicone resin surprisingly improved the wash-off resistance, rub resistance and therefore enhance the deposition of optical particles.

The composition comprising MQ silicone resin, and cholesterol (Sample 2) had the highest deposition ratio increase among these three samples, showing that combination of MQ silicone resin and cholesterol can provide bigger increase for wash-off resistance, rub resistance and the deposition of optical particles.

Table 2

| Sample | Silicone resin-1 (wt% active) | Silicone resin-2 (wt% active) | Silicone resin-3 (wt% active) | Cholesterol (wt% active) | Deposition Ratio (%) |
|---|---|---|---|---|---|
| B | 3.00 | 0 | 0 | 0 | 59.32 |
| C | 0 | 3.00 | 0 | 0 | 36.60 |
| D | 0 | 0 | 1.35 | 0 | 46.15 |
| E | 0 | 0 | 0 | 1.00 | 3.41 |
| 1 | 3.00 | 0 | 0 | 1.00 | 76.29 |

(continued)

| Sample | Silicone resin-1 (wt% active) | Silicone resin-2 (wt% active) | Silicone resin-3 (wt% active) | Cholesterol (wt% active) | Deposition Ratio (%) |
|---|---|---|---|---|---|
| 2 | 0 | 3.00 | 0 | 1.00 | 90.51 |
| 3 | 0 | 0 | 1.35 | 1.00 | 68.39 |

Example 2

This example demonstrates composition with different amount of silicone resin and cholesterol improved the film wash-off resistance, abrasion resistance and therefore deposition of beneficial agent.

The formulations in Table 3 were prepared and the deposition ratios were tested by the following similar procedures as described in Example 1.

Table 3

| Ingredient (wt%) | Sample | | | | | |
|---|---|---|---|---|---|---|
| | 4 | 5 | 6 | 7 | 8 | F |
| Water | Bal. | Bal. | Bal. | Bal. | Bal. | Bal. |
| Disodium EDTA | 0.10 | 0.10 | 0.10 | 0.10 | 0.10 | 0.10 |
| PEG-100 Stearate (Myij 59 P) | 1.85 | 1.85 | 1.85 | 1.85 | 1.85 | 1.85 |
| Glyceryl stearate | 1.00 | 1.00 | 1.00 | 1.00 | 1.00 | 1.00 |
| Caprylic / Capric Triglycerides | 3.00 | 3.00 | 3.00 | 3.00 | 3.00 | 3.00 |
| Aristoflex AVC UL | 1.00 | 1.00 | 1.00 | 1.00 | 1.00 | 1.00 |
| Cyclomethicone/DC 245 | 8.00 | 8.00 | 8.00 | 8.00 | 8.00 | 8.00 |
| Preservative | 0.20 | 0.20 | 0.20 | 0.20 | 0.20 | 0.20 |
| Titanium dioxide* | 1.00 | 1.00 | 1.00 | 1.00 | 1.00 | 1.00 |
| Silicone resin-2* | 3.00 | 3.00 | 3.00 | 2.00 | 1.00 | 0.50 |
| Cholesterol * | 0.05 | 0.10 | 0.30 | 1.00 | 1.00 | 1.00 |
| * Same as in Table 1 | | | | | | |

Table 4 shows the deposition ratios of the compositions with silicone resin and cholesterol with different concentration and weight ratio. The compositions of the present invention (sample 4, 5, 6, 7, and 8) had improved deposition with the addition of cholesterol, showing that the composition comprising silicone resin, and cholesterol with different ratio and/or concentration can improve wash-off resistance, rub resistance and the deposition of optical particles. The composition comprising silicone resin and cholesterol with a weight ratio of silicone resin to cholesterol of 1:2 (sample F) did not shows improved deposition.

Among the compositions of the present invention, both the compositions having silicone resin and cholesterol in a weight ratio of 30:1, 10:1, 3:1 and 2:1 (sample 5, 6, 2 and 7) had a deposition ratio greater that 70%, demonstrating that the performances of wash-off resistance, rub resistance and the deposition of optical particles of compositions having silicone resin and cholesterol in a weight ratio of 30:1, 10:1, 3:1 and 2:1 were better than others.

Table 4

| Sample | Silicone resin (wt% active) | Cholesterol (wt% active) | Weight ratio of silicone resin to cholesterol | Deposition Ratio (%) |
|---|---|---|---|---|
| 4 | 3.00 | 0.05 | 60:1 | 39.34 |
| 5 | 3.00 | 0.10 | 30:1 | 75.08 |
| 6 | 3.00 | 0.30 | 10:1 | 85.14 |

(continued)

| Sample | Silicone resin (wt% active) | Cholesterol (wt% active) | Weight ratio of silicone resin to cholesterol | Deposition Ratio (%) |
|---|---|---|---|---|
| 7 | 2.00 | 1.00 | 2:1 | 74.73 |
| 8 | 1.00 | 1.00 | 1:1 | 50.68 |
| F | 0.50 | 1.00 | 1:2 | 15.94 |

## Claims

1. A cosmetic composition comprising a film-forming polymer having a contact angle of at least 85°, a steroid, and cosmetically acceptable carrier, wherein (i) the weight ratio of film-forming polymer to steroid is at least 5:9; (ii) the carrier is water; (iii) the film-forming polymer is silicone resin; and (iv) the amount of water is 20 to 95% by weight of the composition.

2. The composition according to claim 1 wherein the film-forming polymer is present in amount of from 0.01 to 20% by weight of the composition, preferably from 0.5 to 7%.

3. The composition according to claim 1 or 2 wherein the weight ratio of the film-forming polymer to the steroid is in the range of 3:2 to 35:1.

4. The composition according to any one of the preceding claims wherein the silicone resin comprises MQ silicone resin, T silicone resin, or a mixture thereof.

5. The composition according to claim 5 wherein the silicone resin comprises trimethylsiloxy silicate and/or polypropyl silsesquioxane.

6. The composition according to any one of the preceding claims wherein the silicone resin is present in amount of from 0.01 to 20% by weight of the composition, preferably from 0.5 to 7%.

7. The composition according to any one of the preceding claims wherein the steroid comprises sterol.

8. The composition according to claim 8 wherein the steroid is cholesterol.

9. The composition according to any one of the preceding claims wherein the steroid is present in amount of 0.01 to 10% by weight of the composition, preferably from 0.1 to 3%.

10. The composition according to any one of the claims 4 to 10 wherein the weight ratio of the silicone resin to the sterol is in the range of 3:2 to 35:1.

11. The composition according to any one of the preceding claims wherein the composition comprises optical particle.

12. The composition according to claim 12 wherein the optical particle comprises titanium dioxide, zinc oxide, zirconium oxide, or a mixture thereof.

13. A method for improving skin characteristics selected from skin firming, wash-off resistance, abrasion resistance, cumulative deposition of beneficial agent, long-lasting beneficial agent deposition wherein "long-lasting" refers to the beneficial agent remains at least 30% after flushing by tap water (25°C) for 1 minute, comprising the step of topically applying to skin the composition of any one of the preceding claims, wherein the beneficial agent comprises optical particle, sunscreen agent or a mixture thereof.

14. Use of a composition of any one of claims 1 to 13 for improving any attribute selected from skin firming, wash-off resistance, abrasion resistance, cumulative deposition of beneficial agent, long-lasting beneficial agent deposition wherein "long-lasting" refers to the beneficial agent remains at least 30% after flushing by tap water (25°C) for 1 minute, or combination thereof, wherein the beneficial agent comprises optical particle, sunscreen agent or a mixture

thereof.

## Patentansprüche

1. Kosmetische Zusammensetzung, umfassend ein filmbildendes Polymer mit einem Kontaktwinkel von mindestens 85°, ein Steroid und einen kosmetisch akzeptablen Träger, wobei (i) das Gewichtsverhältnis vom filmbildenden Polymer zu Steroid mindestens 5:9 beträgt, (ii) der Träger Wasser ist, (iii) das filmbildende Polymer ein Silikonharz ist und (iv) die Menge an Wasser 20 bis 95 Gew.-% der Zusammensetzung beträgt.

2. Zusammensetzung nach Anspruch 1, wobei das filmbildende Polymer in einer Menge von 0,01 bis 20 Gewichts-% der Zusammensetzung, vorzugsweise von 0,5 bis 7%, vorliegt.

3. Zusammensetzung nach Anspruch 1 oder 2, wobei das Gewichtsverhältnis des filmbildenden Polymers zum Steroid in dem Bereich von 3:2 bis 35:1 liegt.

4. Zusammensetzung nach irgendeinem der vorhergehenden Ansprüche, wobei das Silikonharz MQ-Silikonharz, T-Silikonharz oder eine Mischung davon umfasst.

5. Zusammensetzung nach irgendeinem der Ansprüche 1 bis 3, wobei das Silikonharz Trimethylsiloxysilikat und/oder Polypropylensilsesquioxan umfasst.

6. Zusammensetzung nach irgendeinem der vorhergehenden Ansprüche, wobei das Silikonharz in einer Menge von 0,01 bis 20 Gewichts-% der Zusammensetzung, vorzugsweise von 0,5 bis 7%, vorliegt.

7. Zusammensetzung nach irgendeinem der vorhergehenden Ansprüche, wobei das Steroid Sterol umfasst.

8. Zusammensetzung nach Anspruch 7, wobei das Steroid Cholesterin ist.

9. Zusammensetzung nach irgendeinem der vorhergehenden Ansprüche, wobei das Steroid in einer Menge von 0,01 bis 10 Gewichts-% der Zusammensetzung, vorzugsweise von 0,1 bis 3%, vorliegt.

10. Zusammensetzung nach irgendeinem der vorhergehenden Ansprüche, wobei das Gewichtsverhältnis des Silikonharzes zu dem Sterol in dem Bereich von 3:2 bis 35:1 liegt.

11. Zusammensetzung nach irgendeinem der vorhergehenden Ansprüche, wobei die Zusammensetzung optische Partikel umfasst.

12. Zusammensetzung nach Anspruch 11, wobei die optischen Partikel Titandioxid, Zinkoxid, Zirconiumoxid oder eine Mischung davon umfassen.

13. Verfahren zur Verbesserung der Hauteigenschaften, ausgewählt aus der Hautstraffung, Abwaschbeständigkeit, Abriebfestigkeit, kumulativer Ablagerung von nützlichem Mittel, langanhaltender Ablagerung von nützlichem Mittel, wobei sich "langanhaltend" auf das nützliche Mittel bezieht, das zu mindestens 30% nach dem Abspülen mit Leitungswasser (25°C) während 1 Minute verbleibt, umfassend den Schritt des topischen Auftragens der Zusammensetzung nach irgendeinem der vorhergehenden Ansprüche auf die Haut, wobei das nützliche Mittel optische Partikel, Sonnenschutzmittel oder eine Mischung davon umfasst.

14. Verwendung einer Zusammensetzung nach irgendeinem der Ansprüche 1 bis 13 zur Verbesserung irgendeiner Eigenschaft, ausgewählt aus der Hautstraffung, der Abwaschbeständigkeit, der Abriebfestigkeit, der kumulativen Ablagerung von nützlichem Mittel, der langanhaltenden Ablagerung von nützlichem Mittel, wobei sich "langanhaltend" auf das nützliche Mittel bezieht, das zu mindestens 30% nach dem Abspülen mit Leitungswasser (25°C) während 1 Minute verbleibt, oder eine Kombination davon, wobei das nützliche Mittel optische Partikel, Sonnenschutzmittel oder eine Mischung davon umfasst.

**Revendications**

1. Composition cosmétique comprenant un polymère formant un film ayant un angle de contact d'au moins 85°, un stéroïde, et un support cosmétiquement acceptable, où (i) le rapport en masse de polymère formant un film au stéroïde est d'au moins 5 : 9 ; (ii) le support est l'eau ; (iii) le polymère formant un film est une résine de silicone ; et (iv) la quantité d'eau est de 20 à 95 % en masse de la composition.

2. Composition selon la revendication 1, où le polymère formant un film est présent dans une quantité de 0,01 à 20 % en masse de la composition, de préférence de 0,5 à 7 %.

3. Composition selon la revendication 1 ou 2, où le rapport en masse du polymère formant un film au stéroïde se trouve dans l'intervalle de 3 : 2 à 35 : 1.

4. Composition selon l'une quelconque des revendications précédentes, où la résine de silicone comprend une résine de silicone MQ, une résine de silicone T, ou un mélange de celles-ci.

5. Composition selon l'une quelconque des revendications 1 à 3, où la résine de silicone comprend du triméthylsiloxysilicate et/ou polypropylsilsesquioxane.

6. Composition selon l'une quelconque des revendications précédentes, où la résine de silicone est présente dans une quantité de 0,01 à 20 % en masse de la composition, de préférence de 0,5 à 7 %.

7. Composition selon l'une quelconque des revendications précédentes, où le stéroïde comprend du stérol.

8. Composition selon la revendication 7, où le stéroïde est le cholestérol.

9. Composition selon l'une quelconque des revendications précédentes, où le stéroïde est présent dans une quantité de 0,01 à 10 % en masse de la composition, de préférence de 0,1 à 3 %.

10. Composition selon l'une quelconque des revendications précédentes, où le rapport en masse de la résine de silicone au stérol se trouve dans l'intervalle de 3 : 2 à 35 : 1.

11. Composition selon l'une quelconque des revendications précédentes, où la composition comprend une particule optique.

12. Composition selon la revendication 11, où la particule optique comprend du dioxyde de titane, oxyde de zinc, oxyde de zirconium, ou un mélange de ceux-ci.

13. Procédé pour l'amélioration des caractéristiques de la peau choisies parmi la fermeté de la peau, la résistance au lavage, la résistance à l'abrasion, le dépôt cumulé d'agent bénéfique, le dépôt d'agent bénéfique sur une longue durée où «longue durée» fait référence à ce que l'agent bénéfique reste pour au moins 30 % après rinçage avec de l'eau courante (25°C) pendant 1 minute, comprenant l'étape d'application topique à la peau de la composition selon l'une quelconque des revendications précédentes, où l'agent bénéfique comprend une particule optique, un agent d'écran solaire ou un mélange de ceux-ci.

14. Utilisation d'une composition selon l'une quelconque des revendications 1 à 13 pour améliorer tout attribut choisi parmi la fermeté de la peau, la résistance au lavage, la résistance à l'abrasion, le dépôt cumulé d'agent bénéfique, le dépôt d'agent bénéfique sur une longue durée où «longue durée» fait référence à ce que l'agent bénéfique reste pour au moins 30 % après rinçage avec de l'eau courante (25°C) pendant 1 minute, ou combinaison de ceux-ci, où l'agent bénéfique comprend une particule optique, un agent d'écran solaire ou un mélange de ceux-ci.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20080233075 A1
- EP 1172138 A2